# EUROPEAN PATENT SPECIFICATION

(11) **EP 2 600 808 B1**
(45) Date of publication and mention of the grant of the patent: **11.12.2013**
(21) Application number: 10763463.6
(22) Date of filing: 03.08.2010
(51) Int. Cl.: A61F 5/02, A61F 5/37

(54) **ORTHOPAEDIC DEVICE**
ORTHOPÄDISCHE VORRICHTUNG
DISPOSITIF ORTHOPÉDIQUE

(43) Date of publication of application: 12.06.2013
(73) Proprietor: Von Zieglauer, Max, 39031 Brunico BZ (IT); Von Zieglauer, Elisabeth, 39031 Brunico BZ (IT)
(72) Inventor: VON ZIEGLAUER, Elisabeth, I-39031 Brunico BZ (IT)
(74) Representative: Marchi, Paolo
(86) International application number: PCT/IT2010/000351
(87) International publication number: WO 2012/017456

(56) References cited:
- EP-A1- 0 966 932
- WO-A1-99/09915
- WO-A1-2009/052031
- US-A- 5 181 906
- US-A1- 2006 129 076

## Description

The present disclosure relates to an orthopaedic device, and in particular to a device adapted to be worn on by a person or patient who, e.g. following an ictus or owing to a hemiplegia, needs a support in order to keep a physiological posture of a shoulder, beside of the spinal column and neck. The orthopaedic device can be employed by a patient also for keeping a physiological posture and attaining healing in case of a collarbone fracture.

Considering, e.g., a hemiplegia, the latter is a paralysis of one half of a person's body and it is typically due to a lesion of the central nervous system at the pyramidal tract. Analogously to hemiparesis, hemiplegia is a motor deficit that can reveal itself in an adult individual following vascular problems, e.g. ictus or haemorrhages, or following traumas, tumours, or due to degenerative and infective causes. Therefore, patients suffering from hemiplegia partially lose mobility of limbs concerned by the lesion.

With a view to preventing degenerative effects of the lesions and fostering, when possible, mobility recovery, it is known the use of orthopaedic devices adapted to impose to limbs a correct posture of shoulder, spinal column and neck, thereby safeguarding the residual functionality of joints, ligaments and muscles.

In case the motor deficit concerns the upper part of the body, and in particular the back and shoulders, orthopaedic devices are known which comprise harnesses adapted to be arranged on the patient's back and constrainable to the trunk at the waist and shoulders by suitable bands and shoulder straps. By suitably tensioning the bands and shoulder straps, it is possible to create on the patient's body a system of "external" forces in the attempt of imposing the correct posture to back and shoulders, which otherwise would tend to sink down and fall forward, i.e., with an inward rotation of the joint, owing to the lack of muscle control and also by effect of the weight of the paralyzed arms.

For instance, patent application EP 0966932 describes an orthopaedic support harness, comprising a strap adapted to be placed on a patient's back and having a length substantially corresponding to the distance between waist and neck and a breadth substantially corresponding to the distance between shoulder blades. To the top portion of the strap two shoulder straps are laterally connected, adapted to wrap the patient's shoulders by passing respectively at the sides of the neck and under the patient's armpits. At the bottom end of the strap, a strap adapted to wrap the patient's waist is connected. Once constrained to the patient's waist and shoulders, the harness allows to support the shoulders, preventing them from falling forward.

Orthopaedic harnesses having features analogous to the abovementioned one are described in US Patents N° 5,135,470 and 5,466,214.

US Patent N° 5,181,906 describes a shoulder joint bandage, comprising an elastic sleeve adapted to fit over a patient's arm and a cap adapted to cover the shoulder. The bandage is provided with belts that can be wound around the sleeve and cap, thereafter contacting the patient's chest and back while extending downwardly and diagonally to the armpit of the opposite arm. The belts further extend from this point, in a horizontal direction, allowing the wrapping of the bandage around the trunk. In the front portion of the cap, a pocket is sewn, adapted to receive a pad capable of applying a pressure to the shoulder joint in order to stabilize it in its motions.

US patent application N° US 2006/0129076 discloses a broken collar bone fixing band.

International patent application publication N° WO 99/09915 discloses a multi-support for correction of subluxed shoulder.

International patent application publication N° WO 2009/052031 discloses an adjustable posterior spinal orthosis.

Object of the present disclosure is to provide improving solutions in this field, in particular concerning the effective keeping of a physiological position of the shoulder joint of a patient, and/or to attain further advantages with respect to the known art.

In particular, an orthopaedic device according to claim 1 and an orthopaedic kit according to claim 15 are provided. Secondary features of the subject of the present disclosure are set forth in the corresponding dependent claims.

Basically, an idea of solution at the basis of the present disclosure is that of associating, to an orthopaedic harness provided with a backpiece portion constrainable to a patient's trunk and at least one shoulder strap, a support pad adapted to be inserted under the patient's armpit to exert an action opposing motions of external and internal rotation of the joint of the shoulder suffering from disease, as well as an action of supporting the joint itself; in particular, the support pad is shaped in a way substantially matching the axillary cavity, thereby allowing an adequate action of supporting and centring the articulation or glenohumeral joint.

In other words, the support pad is the "negative" of the axillary cavity, i.e., it has a shape substantially complementary to the shape of the latter.

The orthopaedic harness is basically a bandaging or bandage capable of keeping the support pad in position: the interaction between the orthopaedic harness and the patient's body to which it is constrained creates a system of lifting forces acting onto the support pad.

The backpiece portion (which does not necessarily have to cover the entire back of the patient; on the contrary, it can be arranged at a limited region of the back itself) is constrainable to the patient's body by suitable fastening means, like, e.g., an abdominal belt or strap.

The orthopaedic device according to the present disclosure allows a patient, who following a paralysis or an ictus has lost control of the muscles of a shoulder, as well as of the chest and an arm, to keep a position as physiological as possible, with a centering of the humeral head of the shoulder joint itself. Therefore, the risk of painful degenerations of the joint (which can lead to crooked spinal column and neck) is abated, and a position more favourable for the restoring of functional movement is guaranteed to the concerned upper limb.

Likewise, the orthopaedic device according to the present disclosure allows a patient who has had a collarbone fracture to keep a position as physiological as possible, enabling a correct forming of the bone callus, and thereafter a faster functional rehabilitation.

The support pad makes possible the lifting of the shoulder and its keeping into a physiological position, a keeping which therefore is not reached merely by shoulder strap traction: in fact, a synergistic effect between shoulder strap and support pad is attained. Thus, a selective lifting of the shoulder is obtained, which is generated not merely by traction force, but also by the upward-pushing action exerted by the pad. Thanks to the shape matching the axillary cavity, the push of the pad is well-distributed on the wall of the axillary cavity and it complies with the anatomy of the joint. This also gives the patient an increased feeling of safety, as the shoulder is kept on the inside by the support pad and on the outside by the ring formed by the shoulder strap.

All components of the orthopaedic device are designed to minimize the consequences of hemiplegia on the patient. Their shape and arrangement is such that pathological effects on the vascular / nervous system, on the joint capsule, on ligaments and on shoulder zone muscles are not worsened. Moreover, the materials used guarantee to the patient a remarkable comfort, which also reduces allergic reactions of the skin.

Preferably, the shoulder strap is fastened on the backpiece portion in a manner such that, in an extended configuration of the harness (i.e., when the latter is flattened on a plane), the shoulder strap and the backpiece portion form a preset angle therebetween, so as to allow a positioning of the shoulder joint in the most physiological manner, thereby avoiding a dangerous deformity or a subluxation. A suitable selection of the angle allows to cause the shoulder strap to pass externally to the shoulder (basically, at the top of the arm), and therefore be more effective at drawing the shoulder backwards toward the back. In particular, it is important to keep the shoulder straight with respect to the spinal column, so that the latter as well may remain straight and undeformed. In fact, other physical problems, even very serious ones, may occur in case of spinal column deformation: ligaments and muscles shrink and lose tone, nerves get stuck and vertebral discs are compressed. The consequences of this can be serious and entail, e.g., migraine or disc herniation.

In one embodiment, the support pad, which is associated with the shoulder strap wrapping the injured shoulder of the patient, may advantageously be removably coupled with the shoulder strap of the harness, thereby allowing its replacement and adjustment to the shape and size of the patient's axillary cavity, without entailing a replacement of the entire harness. For instance, it is possible to provide an orthopaedic kit comprising an orthopaedic device according to the present disclosure and a set of support pads different each other in shape and/or size; the support pads are interchangeable with each other on the shoulder strap and the patient can therefore choose to use the one which is best suited to his/her anatomical configuration.

According to one embodiment, the support pad is supported by a mat element allowing an improved distribution of contact pressures generated on tissues and joints by the shoulder strap of the harness. This allows to offer a valid solution to the problem of the compression generally exerted by prior-art devices, and in particular by their shoulder straps, on blood vessels and nerve endings in the shoulder area compression which may cause secondary lymphedemas, localized pain syndromes and, more generally, a worsening of the neurological symptoms, with an increase in the formation of contractures and spasticity.

Specifically, the mat element is arranged between the support pad and the respective shoulder strap and, even more specifically, it has a first face intended to be facing the user's armpit and on which the support pad is mounted, and a second face, opposite to the first face, facing the shoulder strap.

Preferably, the mat element has a breadth greater than the shoulder strap on which it is mounted; thus, it prevents arm nerve cutting and/or excessive compression. This is particularly important since the patient, in case of disorders, loss of sensitivity or cognitive deterioration, is not always able to judge whether the shoulder strap is overly tensioned or excessively tight on the shoulder. The length of the mat element is lower than that of the shoulder strap and is selected so as to effectively distribute the pressure exerted by the shoulder strap.

In one embodiment, the mat element is made of a material having a lattice structure in which an oily substance (e.g., a mineral or silicone oil) is included. The oily substance guarantees softness and good distribution of pressure, i.e., allows distribution and dissipation of forces acting onto the mat element from all directions. Moreover, it provides the further advantage of having a cooling effect; this constitutes an important aspect in case of patients affected by ictus, owing to higher sweat production in the context of a vegetative dysregulation.

In one embodiment, the support pad is removably mounted on the mat element, so that, as already mentioned above, it may easily be replaced to adapt the orthopaedic device to the patient's needs. In particular, it is fastened to the mat element through a hook-and-loop fastening system, like, e.g., Velcro®.

In one embodiment, the mat element is slidably mounted on the shoulder strap; this allows, when the device is worn on by a user, to tension the shoulder strap, tightening around the shoulder with a desired force, concomitantly without having the mat element move along with the shoulder strap and shift from the position in the axillary cavity or deform. Basically, the mat element remains stationary as the shoulder strap slides with respect thereto to tighten around the shoulder; in addition, by not being deformed, the mat element does not change its thickness.

For instance, this is obtained thanks to a sleeve portion which is associated with the mat element and slidably receives the shoulder strap.

Advantageously, the side wall of the sleeve portion is provided with a plurality of window openings that facilitate insertion of the shoulder strap through the sleeve portion, allowing the user to pull the shoulder strap even when the latter is only partially inserted in the sleeve portion.

In one embodiment, the mat element is advantageously associated with one or more rod-shaped transversal stiffener elements, which, by extending across the axillary cavity (i.e., between the torso and the arm) oppose the folding and rolling of the mat element during arm motions; thus, the support pad is kept in a centred position with respect to the glenohumeral joint and the distribution action for contact pressures of the shoulder strap is ensured over a broad surface of the mat element.

In one embodiment, the support pad and the mat element may be advantageously associated into a single module that can be mounted on the shoulder strap of the harness. For this purpose the pad is fastened on the mat element, obtaining an assembly that is inserted into a respective sheathing or casing.

Mounting of the sheathing on the shoulder strap of the harness is preferably carried out through said sleeve portion obtained or fastened on the sheathing itself. This type of mounting slidably constrains the sheathing to the shoulder straps and allows to prevent deformation of the support pad, and of the mat element, during a tensioning of the shoulder straps of the harness.

To further increase fastening stability of the orthopaedic device on the patient's body (i.e., harness stability on the patient's body), which can strongly influence the keeping of a correct posture, a second shoulder strap may be provided, it also associated with the backpiece portion and adapted to wrap the other shoulder of the user, and/or a belt portion associated with the backpiece portion and adapted to wrap the user's waist. The second shoulder strap, i.e., that on the healthy shoulder, does not require to be tightly tensioned like the first shoulder strap for the injured shoulder; however, it is required thai a tension such as to block any incorrect and non-physiological position be applied. To prevent excessive compression on the healthy shoulder, the second shoulder strap is preferably provided with a second mat element which is associated with the second shoulder strap and is intended to be facing the user, i.e., to be interposed between the second shoulder strap and the healthy shoulder. Also this second mat element is preferably removable.

The belt portion, or abdominal support, is advantageous for ensuring that, besides a specific support to the injured shoulder, all of the upper part of the patient's body has a greater stability of the shoulder zone and therefore a more effective positioning of the shoulder or the glenohumeral joint.

In case it is required to support both shoulders (e.g., in case the patient is affected by a paralysis on both sides) a second support pad may be associated with the second shoulder strap with modes and variants analogous to those for the first shoulder strap.

Further advantages, features and operation modes of the orthopaedic device which is the subject of the present disclosure will be made evident to those skilled in the art by the following detailed description of embodiments thereof, given by way of example and not for limitative purposes.

However, it is evident how each embodiment may have one or more of the advantages listed above; in any case, however, it is not required for each embodiment to concomitantly have all of the advantages listed.

It is also understood that all viable combinations of the embodiments indicated above and of those described with reference to the following detailed description fall within the scope of the present disclosure.

Reference will be made to the figures of the annexed drawings, wherein:
- Fig. 1 shows an embodiment of an orthopaedic device according to the present disclosure, in an extended configuration thereof;
- Figs. 2 and 3 schematically show a front view and a rear view, respectively, of the orthopaedic device of Fig. 1 worn on by a patient;
- Fig. 4 shows a partially sectional view of a first face of an assembly comprising a mat element and a support pad according to the present disclosure, in which the parts are partially detached;
- Fig. 5 shows a partially sectional view of a second face, opposite to the first face, of the assembly of Fig. 4, in which the parts are partially detached;
- Fig. 6A shows a plan view of a first embodiment of a support pad according to the present disclosure;
- Fig. 6B shows a side view of the support pad of Fig. 6A;
- Fig. 7A shows a plan view of a second embodiment of a support pad according to the present disclosure;
- Fig. 7B shows a side view of the support pad of Fig. 7A; and
- Fig. 8 shows a perspective view (with isolines to highlight the curvatures) of a set of support pads according to the present disclosure.

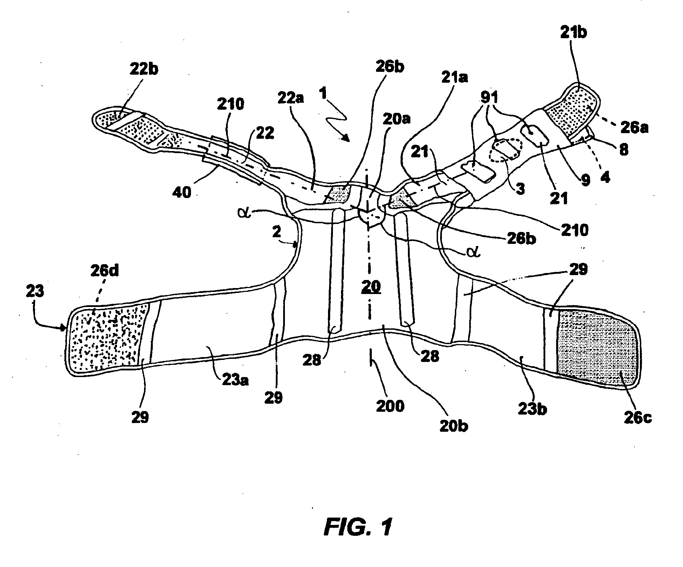

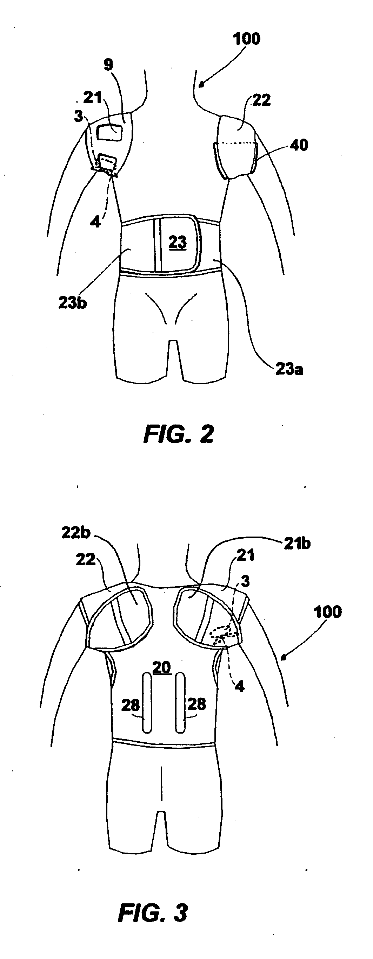

Referring initially to Figs. 1 to 3, an orthopaedic device according to the present disclosure is denoted by reference number 1. In particular, the orthopaedic device 1 finds application in the rehabilitation of a user or patient 100 suffering from hemiplegia or similar pathology causing a dysfunction in the joint region of an upper limb, e.g. owing to paralyzed muscles, or in the rehabilitation of a patient 100 who has had a collarbone fracture.

The orthopaedic device 1 comprises a harness 2 provided with a back or backpiece portion 20, which is adapted to be placed or arranged on the back of a user or patient 100 and has a length substantially corresponding to the distance between waist and neck of the user 100 and a breadth substantially corresponding to the distance between shoulder blades. The backpiece portion 20 may be provided with a plurality of stiffener elements 28, e.g. rod- or strip-shaped, which are longitudinally arranged, i.e. develop along the waist-neck direction, and are adapted to foster an action supporting the back of the patient 100, also preventing the backpiece portion 20 from rolling up or folding toward the neck.

The stiffener elements described in the present disclosure have a certain flexibility, which however is very limited with respect to the flexibility of the fabric or member to which they are applied. Therefore, the stiffener elements allow to keep the fabric taut and to prevent that it, by rolling up, may decrease the surface of contact with the user 100; concomitantly, they however allow some bending of the fabric, to conform to the user's body and comply with his/her motions within certain extents.

Two shoulder straps or belts 21, 22 are laterally associated with an upper or top end 20a of the backpiece portion 20; the shoulder straps 21, 22 extend from opposite sides with respect to a longitudinal axis of symmetry 200 of the backpiece portion 20 and are suitably sized to wrap each a respective shoulder of the patient 100, passing aside the neck and below the respective armpit, to close up on the backpiece portion 20. In other words, a first end 21a, 22a of each shoulder strap 21, 22 is fastened, e.g. by sewing, to the upper part 20a of the backpiece portion 20, whereas a second end 21b, 22b of each shoulder strap 21, 22 can be removably fastened to the backpiece portion 20 so that the shoulder strap 21, 22, by assuming a ring-like shape, surrounds and wraps round the respective shoulder of the user 100. The fastening points 26b of the second ends 21b, 22b of the shoulder straps 21, 22 on the backpiece portion 20 are, in the example, near the respective first ends 21a, 22a of the shoulder straps 21, 22 themselves, in a way such that the latter form said ring.

When the orthopaedic device 1 is worn on by the patient 100, each shoulder strap 21, 22 is passed over the respective shoulder, on the front portion of the shoulder and below the axillary cavity, projecting in the back region. The second end 21b, 22b is pulled so as to tension the shoulder strap 21, 22 itself and tighten on the shoulder; the second end 21b, 22b is finally fastened to the respective fastening point 26b on the backpiece portion 20.

Basically, the harness 2 implements a bandaging or bandage of a body part of the user 100.

The fastening of the second end 21b, 22b of the shoulder straps 21, 22 on the backpiece portion 20 is preferably of hook-and-loop type, e.g. comprising Velcro® strips 26a, 26b, so as to simply and economically allow the adjustment of the fastening point of the second end 21b, 22b, and therefore of the tension and strength with which the shoulder strap 21, 22 tightly wraps the respective shoulder. Preferably, the Velcro® strips 26a at the second ends 21b, 22b extend to about one-third of the length of the respective shoulder strap 21, 22, so as to have a greater option of stepless adjustment (i.e., an adjustment having a fastening without fixed positions at preset intervals) and also a greater adaptability to different patients.

Alternatively, other fastening means well-known to those skilled in the art, e.g. buckles or buttons, may be used.

The second ends 21b, 22b of the shoulder straps 21, 22 may be cut to measure by an orthopaedic technician or a physiotherapist, in case it is necessary to adapt the orthopaedic device 1 to the patient 100, e.g., when the shoulder straps 21, 22 are too long. Thanks to the material used, this may be done without shoulder strap laddering.

It will be noted that, in the extended configuration of the harness 2 shown in Fig. 1, the shoulder straps 21, 22 form a preset angle α with the backpiece portion 20, which in the example is of 45 degrees. As shown in Fig. 1, the angle α is, e.g., defined between the longitudinal axis 200 of the backpiece portion 20 and the longitudinal axis 210 of the respective shoulder strap 21, 22.

The selection of a suitable value for the angle α allows to confer a greater comfortableness and wearability of the harness 2, both in a resting position and during motion. Moreover, it allows an adaptation of the shoulder straps 21, 22 and of the backpiece portion 20 to the back in a close-fitting way, conferring remarkable stability to the harness 2 once it is worn on by the patient 100. The shoulder strap 21, 22 effectively blocks the shoulder and minimizes the onset of undesired relative motions between the different portions of the joint, which relative motions might penalize the shoulder-supporting action. Therefore, the selection of angle α is important for shoulder blocking, comfort and the patient's feeling of safety.

Preferably, during the manufacturing of the orthopaedic device 1 the upper end region 20a of the backpiece 20 onto which the shoulder strap 21, 22 is sewn is subjected to traction toward the top, i.e., it is pulled and deformed substantially parallel with the longitudinal axis 200. Said region is kept taut (preferably with an angular deformation) during the sewing of the first end 21a, 22a of the shoulder strap 21, 22.

For instance, prior to the carrying out of the sewing of a shoulder strap 21, 22, a wedge of material having a 19° angle is cut away from the upper region 20a of the backpiece 20; the edge thus created is stretched to recover the removed wedge surface and is kept taut during the sewing. Upon completing the sewing, elastic return of the previously stretched portion drags therewith the opposite edges of the first end 21a, 22a of the respective shoulder strap 21, 22; accordingly, the first end 21a, 22a of the shoulder strap 21, 22 slightly cambers in the direction of its breadth, assuming a shape that follows the anatomical curvature of the shoulder and back, therefore forming a strap having an anatomical shape. Hence, when the orthopaedic device 1 is in an extended configuration flattened on a plane, said sewing region of the backpiece portion 20 is in a traction-tensioned condition.

Moreover, the backpiece portion 20 is provided, at the lower or bottom end 20b, with a belt portion 23 adapted to be wrapped around the waist of the patient 100. In particular, the belt portion 23 comprises two straps 23a, 23b extending from opposite sides of the backpiece portion 20, wherein the free ends of the straps 23a, 23b may be fastened to each other (e.g., at the abdomen of the patient 100) preferably by a removable hook-and-loop fastening, e.g. comprising Velcro® strips 26c, 26d. The belt portion 23 therefore allows to constrain the backpiece portion 20 to the waist or midriff of the patient 100. Preferably, the Velcro® strip 26d at the free end of the strap 23a extends to about one-third of the length of the respective strap 23a, so as to have a greater adjustment option and a greater adaptability to different patients.

As an alternative, other fastening means well-known to those skilled in the art, e.g. buckles or buttons, may be used.

The belt portion 23 as well is preferably provided with a plurality of stiffener elements 29, e.g. rod- or strip-shaped, which are arranged transversally to the straps 23a, 23b to prevent the latter from rolling up or folding; thus, it is prevented that the straps 23a, 23b, by rolling up in the breadth direction, reduce their breadth and the surface of contact with the patient 100.

Backpiece portion 20, shoulder straps 21, 22 and belt portion 23 are preferably made of a breathable fabric having a certain degree of elasticity. For instance, such a fabric is of interlock type, and it is an elastic fabric with 80% cotton and 20% elastan, having a 50-60% elasticity and a density of 470 grams per square meter. In particular, the shoulder straps 21, 22 are made of elastic plush, and a padding may be provided to improve contact pressure distribution.

Once the shoulder straps 21, 22 and the belt portion 23 are fastened and suitably tensioned, the harness 2 and in particular the backpiece portion 20 are firmly constrained to the body of the patient 100 and exert a plurality of forces on the patient's trunk, allowing to keep the back and the shoulders in a substantially erect position without hindering motions of shoulders and arms.

As discussed above, a hemiplegic patient usually has a motor deficit involving one half of the body (in this case, one half of the chest) and typically the shoulder and arm. The supporting action exerted by the orthopaedic device 1 according to the present disclosure is directed in particular to the shoulder-arm complex, and also to the spinal column; for a greater effectiveness, the orthopaedic device 1 further comprises a pad 3 associated with a shoulder strap and intended to support the injured shoulder of the patient 100. Referring to the embodiment shown in Fig. 1, the support pad 3 is associated with the shoulder strap denoted by reference number 21.

The support pad 3 has a shape substantially matching the shape of the axillary cavity of the injured shoulder and is arranged at the face of the shoulder strap 21 that is intended to be facing the axillary cavity. In other words, the support pad 3 is substantially complementary to the axillary cavity.

In fact, the support pad 3 is intended to be housed in the respective axillary cavity of the user 100, to keep the shoulder joint in a preset physiological position in which the humeral head is centred in the glenohumeral joint. Basically, correspondence between the shape of the support pad 3 and the shape of the axillary cavity allows to exert on the joint a thrust that is adapted to keep the glenohumeral joint centred, effectively opposing the extra- and intra-rotation motions that the patient is no longer able to control due to the injury or ictus.

When the orthopaedic device 1 is worn on by a patient 100, the shoulder strap 21 is positioned and tensioned so that the support pad 3 is pressed in the axillary cavity and pushes against the walls of the latter: therefore, the joint is kept in the physiological position that a healthy person would have, in particular it is pushed upward and toward the back; thus, a degeneration of the state of the joint is prevented and functional recovery of the latter is fostered.

According to an aspect of the invention, the support pad 3 is removably associated with the shoulder strap 21, thereby allowing its replacement without requiring a replacement of the entire harness 2.

The support pad 3 is preferably associated with the shoulder strap 21 in a removable manner, e.g. through a hook-and-loop fastening comprising Velcro® strips, thereby simply and economically allowing a fine adjustment of the position of the support pad 3 on the shoulder strap 21 and an easy replacement thereof.

The shape and size of the support pad 3 to be used depend on the shape and size of the specific axillary cavity of the patient 100. In particular, it is possible to envisage providing a set of support pads 3, i.e. a plurality of support pads 3 different each other in shape and/or size: the user 100 selects among said plurality of support pads the pad 3 which is best suited to him/her, and associates it to the shoulder strap 21. In fact, thanks to the removable fastening, the support pads 3 are interchangeable with each other on the shoulder strap 21.

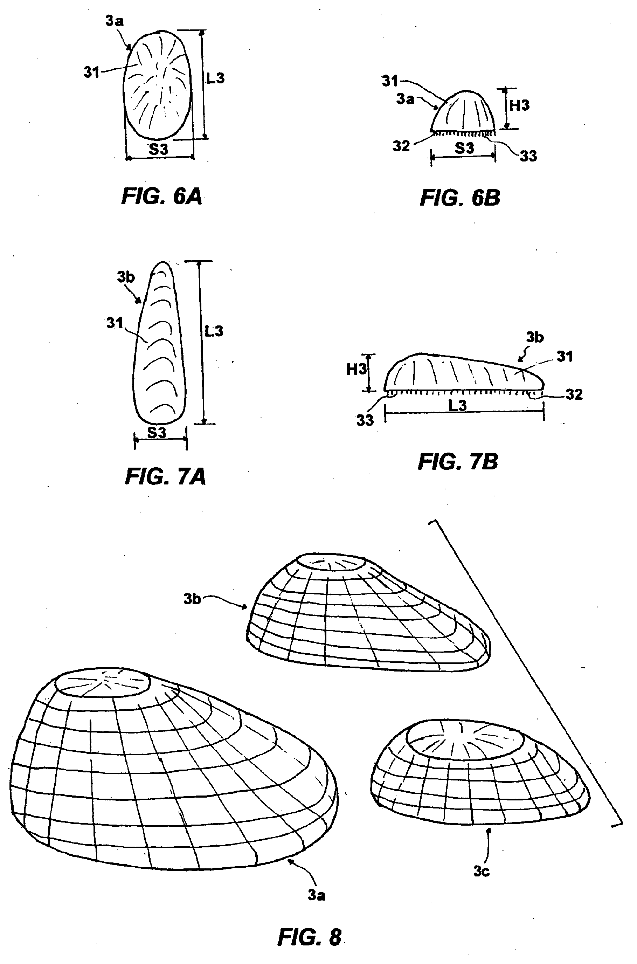

By way of example, a first embodiment of a support pad 3a is shown in Figs. 6A, 6B and 8, a second embodiment of a support pad 3b is shown in Figs. 7A, 7B and 8, a third embodiment of a support pad 3c is shown in Fig. 8.

In the example, the first embodiment of the support pad 3a (Figs. 6A, 6B and 8) has an oval-plan cusp shape, having a length L3 of 8 cm, a thickness S3 of 5.5 cm and a maximum height H3 of 3.5 cm.

In the example, the second embodiment of the support pad 3b (Figs. 7A, 7B and 8) has a lanceolate-plan cusp shape, having a length L3 of 7 cm, a thickness S3 of 4 cm and a maximum height H3 of 3 cm.

In the example, the third embodiment of the support pad 3c (shown in Fig. 8 only) has a length L3 of 5 cm, a thickness S3 of 3 cm and a maximum height H3 of 1.8 cm.

By way of illustration, Fig. 8 shows a set of three support pads 3a, 3b, 3c, different each other in shape and size, which are interchangeable on the shoulder strap 21.

In the embodiments shown, the support pad 3 has a top face 31 which is intended to be facing the wall of the axillary cavity, having a curved shape substantially corresponding to the latter, and a bottom face 32 which, e.g., is flat and provided with fastening means 33 of hook-and-loop type.

In the example, the support pad 3 is made of PX foam or open-cell polyurethane, of the type that is also used for anti-decubitus cushions for diabetic patients.

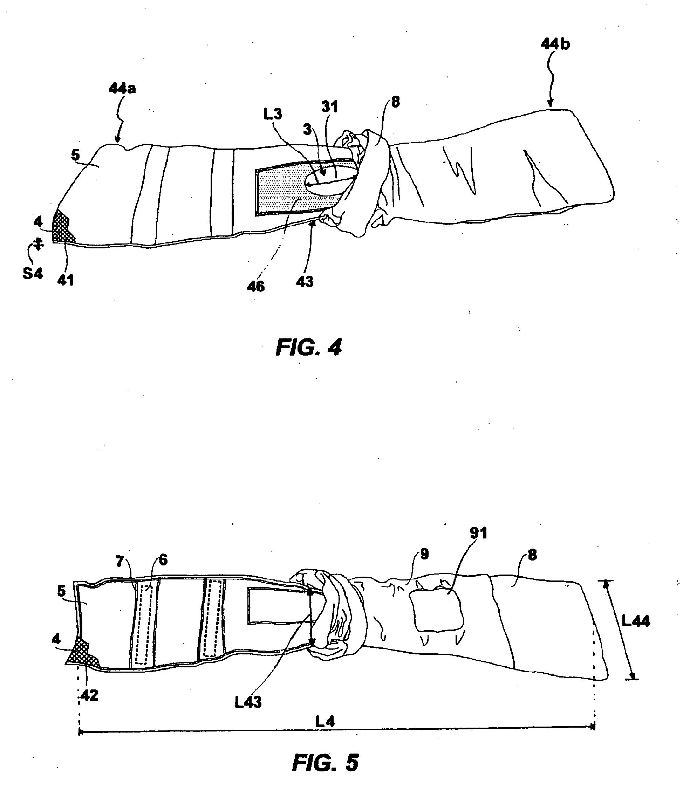

Specifically referring to Figs. 4 and 5, the orthopaedic device 1 may further comprise a mat element 4 associated with the support pad 3. The mat element 4 is interposed between the support pad 3 and the shoulder strap 21; in particular, the mat element 4 has a first face 41 intended to be facing the armpit of the patient 100 and on which the support pad 3 is mounted, and a second face 42, opposite to the first face 41, which is facing the shoulder strap 21.

In other words, the second face 42 of the mat element 4 is adjacent to the face of the shoulder strap 21 which is intended to be facing the armpit of the patient 100, whereas the support pad 3 is placed adjacent to the first face 41 of the mat element 4 intended to be facing the armpit of the patient 100. Therefore, by tensioning the shoulder strap 21, the latter presses against the mat element 4, which in turn presses the support pad 3 into the patient's axillary cavity. Accordingly, the support pad 3 is pushed upward, to support the shoulder joint in order to keep the mentioned physiological position.

The main function of the mat element 4 is that of distributing contact pressures generated by compression forces exerted by the shoulder strap 21 onto the patient's armpit. Thus, thank to an uniform distribution of the contact pressures on a surface greater than that of the sole support pad 3 and the portion of the shoulder strap 21 supporting it, it is possible to prevent the formation of lymphedemas, localized pain syndromes and, more generally, to oppose as much as possible phenomena of worsening of the neurological symptoms, which might lead to contracture formation and spasticity.

The mat element 4 is made of a layer of light-weight, anergic and flexible material. Preferably, the mat element 4 has a lattice structure of polymeric material, in which an oily substance is included (e.g., a mineral or silicone oil), ensuring the distribution and dissipation of forces acting in a vertical or horizontal direction; concomitantly, the oily substance moisturizes and nourishes the patient's skin.

In the example, said layer of material has a thickness S4 of 4 mm.

Moreover, the mat element 4 has an anatomical shape, in particular a hourglass shape, facilitating its positioning below the armpit without hindering patient's arm motions, and causing venous vessels and nerves to be not compressed also in a resting position. The narrowing portion 43 of the hourglass shape of the mat element 4, i.e. that having a smaller transversal dimension, is housed at the armpit of the patient 100, whereas end portions 44a, 44b rest respectively on the front part and on the rear part of the shoulder, pressed by the shoulder strap 21 of the harness 2. Thus, an effective flat-plane "closure" with the backpiece portion 20 is obtained.

The mat element 4 in the example has a length L4 of 47 cm, a breadth L44 of 11 cm in the (wider) end portion 44a, 44b and a breadth L43 of 8 cm in the (narrower) central or narrowing portion 43.

In addition, the mat element 4 is preferably coated by a protective sheath 5, made for instance of cotton or silk, adapted to prevent its damaging, due e.g. to rubbing against the shoulder strap 21. In Figs. 4 and 5 such a sheath 5 is depicted as partially interrupted to show a corner of the mat element 4.

Moreover, the mat element 4 may be provided with a plurality of stiffener elements 6, e.g. rod- or strip-shaped, arranged in a transversal direction of the mat element 4, i.e. perpendicularly to the direction of insertion of the mat element 4 under the armpit of the patient 100. When the patient 100 wears on the orthopaedic device 1, the transversal stiffener elements 6 rest on the one side against the trunk, and on the other side against the inside of the arm, actively contributing to the centring of the glenohumeral joint and, by preventing the rolling up of the mat element 4, ensuring that all of the transversal breadth of the mat element 4 be operating to redistribute contact pressures.

Advantageously, the transversal stiffener elements 6 may be inserted into suitable pockets 7 made or fastened, e.g. by sewing, on the sheath 5.

As shown in Fig. 4, the support pad 3 is removably mounted on the mat element 4, preferably through hook-and-loop fastening means such as Velcro® elements 33, 46, thereby allowing, as discussed above, the replacement and a fine adjustment of the position of the former.

To foster handling and mounting of the assembly comprising the mat element 4 and the support pad 3, as well as to facilitate their correct positioning in the axillary cavity, said assembly is preferably inserted in a sheathing or casing 8, which therefore encloses the assembly and protects it from soiling and abrasion.

In Figs. 4 and 5 the sheathing 8 is depicted partially unstitched and rolled up, so as to show the assembly enclosed thereby; it is however understood that, in use, the sheathing 8 covers all the assembly (and therefore also the left-hand half of Figs. 4 and 5).

The materials utilized for the manufacturing of sheath 5 and sheathing 8 preferably have antibacterial characteristics; for instance, the sheathing 8 is made of a fabric comprising threads of silver, which is odourless and antibacterial. Moreover, the sheathing 8 is removable and washable. In one embodiment, the material of the sheathing 8 is a knitted fabric thus constituted: 55% cotton, 30% nylon, 10% elastan, 5% raw silver; it is an anti-sweat and antibacterial material.

To allow mounting of the assembly comprising the mat element 4 and the support pad 3 on the shoulder strap 21, the sheathing 8 is advantageously provided with a sleeve portion 9 on the side opposite to that intended to be facing the armpit of the patient 100, said sleeve portion 9 being e.g. fastened externally to the sheathing 8.

The sleeve 9 is adapted to slidably receive the shoulder strap 21 of the harness 2. Basically, the shoulder strap 21 is inserted into said sleeve portion 9, thereby making a slidable connection between the mat element 4 and the shoulder strap 21.

In fact, this type of mounting constrains the sheathing 8, and therefore the mat element 4 and the support pad 3, to the shoulder strap 21 in a slidable manner, and allows to finely adjust the position thereof even once the shoulder strap 21 is tensioned; this has also the purpose of preventing undesired deformations of the mat element 4.

When the orthopaedic device 1 is worn on by the patient 100, the shoulder strap 21 is pulled in order to fasten its second end 21b to the Velcro® portion 26b; the shoulder strap 21 slides inside the sleeve portion 9 and therefore said assembly can remain stationary below the armpit of the patient 100, regardless of how much of the shoulder strap 21 is returned to the back of the patient 100.

Preferably, the side wall of the sleeve portion 9 is provided with window openings 91 allowing access to the shoulder strap 21 received in the sleeve 9. Such openings 91 are advantageous during the insertion of the shoulder strap 21 into the sleeve portion 9, as they allow a user to insert a hand and grab the shoulder strap 21 to pull and slide it into the sleeve 9, even when the shoulder strap 21 is only partially inserted into the sleeve 9 itself.

The second shoulder strap 22, i.e. that lacking the support pad 3, may be provided with a small protector or a mat element or the like, to distribute pressure and/or prevent the shoulder strap 22 from irritating the armpit. E.g., such a protector is a mat element 40 (made of a material analogous to that of the mat element 4 of the first shoulder strap 21) which is fastened to the second shoulder strap 22 with Velcro® or other fastening means. When the orthopaedic device 1 is worn on, such a second mat element 40 is interposed between the second shoulder strap 22 and the body (in particular the shoulder/armpit) of the patient 100. Also such a second mat element 40 is removable.

The subject of the present disclosure has been hereto described with reference to a preferred embodiment thereof. However, it is understood that variants and modifications to said embodiment may be effected, all falling however within the concept of the same invention.

In a possible variant, the backpiece portion 20 is fastened to the body of the user 100 with modes different from the ones described hereto. For instance, a single shoulder strap 21, provided with support pad 3 and intended to support an injured shoulder, might be provided, whereas the backpiece portion 20 is constrained to the body of the user 100 by a plurality of straps surrounding the chest of the user 100. Basically, the abdominal belt portion 23 and/or the second shoulder strap 22 might not be present.

In another variant, the backpiece portion also extends in the breast and abdomen part, thereby substantially defining a corsage.

In yet another variant, the orthopaedic device comprises a corset-shaped portion (e.g. made of a non-elastic fabric and with rigid ribs) which covers the entire abdomen, down to the pubic bone.

Therefore, it is understood that other embodiments might exist, all falling within the concept of the same invention, and all comprised within the protective scope of the claims hereinafter.

## Claims

1. An orthopaedic device (1) comprising:
- a harness (2) provided with a backpiece portion (20) adapted to be arranged on the back of a user (100),
- at least one shoulder strap (21) associated with said backpiece portion (20) and adapted to wrap a respective shoulder of the user (100), and
- a support pad (3, 3a, 3b, 3c) adapted to be associated with said at least one shoulder strap (21),
**characterized in that**
said support pad (3, 3a, 3b, 3c) is intended to be housed in a respective axillary cavity of the user (100) for keeping the shoulder joint in a preset physiological position, wherein said support pad (3, 3a, 3b, 3c) has a shape matching the shape of the axillary cavity.

2. The orthopaedic device (1) according to claim 1, further comprising a mat element (4) interposed between the support pad (3, 3a, 3b, 3c) and the shoulder strap (21), wherein the support pad (3, 3a, 3b, 3c) is mounted on said mat element (4).

3. The orthopaedic device (1) according to claim 2, wherein the support pad (3, 3a, 3b, 3c) is removably mounted on the mat element (4), in particular through hook-and-loop fastening means.

4. The orthopaedic device (1) according to claim 2 or 3, wherein the mat element (4) has a first face (41) intended to be facing the armpit of the user (100) and on which the support pad (3, 3a, 3b, 3c) is mounted, and a second face (42), opposite to the first face (41), which is facing the shoulder strap (21).

5. The orthopaedic device (1) according to any one of claims 2 to 4, wherein the mat element (4) is slidably mounted on the shoulder strap (21), said mat element (4) being mounted on the shoulder strap (21) by means of a sleeve portion (9) which is adapted to slidably receive said shoulder strap (21).

6. The orthopaedic device (1) according to claim 5, wherein a side wall of said sleeve portion (9) is provided with a plurality of window openings (91).

7. The orthopaedic device (1) according to any one of claims 2 to 6, wherein an assembly comprising the mat element (4) and the support pad (3, 3a, 3b, 3c) is enclosed by a casing (8).

8. The orthopaedic device (1) according to any one of claims 2 to 7, comprising a plurality of transversal stiffener elements (6) associated with the mat element (4), the mat element (4) being coated by a protective sheath (5) and the transversal stiffener elements (6) being inserted into respective pockets (7) made on said protective sheath (5).

9. The orthopaedic device (1) according to any one of claims 2 to 8, wherein the mat element (4) is made of a material having a lattice structure in which an oily substance is included.

10. The orthopaedic device (1) according to any one of claims 1 to 9, wherein the support pad (3, 3a, 3b, 3c) is removably associable with the shoulder strap (21).

11. The orthopaedic device (1) according to any one of claims 1 to 10, wherein the shoulder strap (21) is fastened to the backpiece portion (20) by sewing and wherein, in an extended configuration of the harness (2), the shoulder strap (21) forms a preset angle (α) with the backpiece portion (20) and the sewing region of the backpiece portion (20) is in a traction-tensioned condition.

12. The orthopaedic device (1) according to any one of claims 1 to 11, further comprising a belt portion (23) associated with said backpiece portion (20) and adapted to wrap the waist of the user (100).

13. The orthopaedic device (1) according to any one of claims 1 to 12, further comprising a second shoulder strap (22) associated with said backpiece portion (20) and adapted to wrap a second shoulder of the user (100).

14. The orthopaedic device (1) according to claim 13, comprising a second mat element (40) associated with the second shoulder strap (22) and intended to be interposed between the second shoulder strap (22) and the body of the user (100).

15. An orthopaedic kit, comprising an orthopaedic device (1) according to any one of claims 1 to 14 and a set of support pads (3a, 3b, 3c) different from each other in shape and/or size, said support pads (3a, 3b, 3c) being interchangeable with each other on said at least one shoulder strap (21).

## Patentansprüche

1. Orthopädische Vorrichtung (1), umfassend:
- eine Stützbandage (2) mit einem Rückenteilabschnitt (20), der derart ausgebildet ist, dass er auf dem Rücken eines Benutzers (100) anordbar ist,
- zumindest einen Schultergurt (21), welcher mit dem Rückenteilabschnitt (20) verbunden ist, und derart ausgebildet ist, dass er eine entsprechende Schulter des Benutzers (100) umgibt, und
- ein Stützkissen (3, 3a, 3b, 3c), das derart ausgebildet ist, dass es mit dem zumindest einen Schultergurt (21) verbindbar ist,
**dadurch gekennzeichnet,**
**dass** das Stützkissen (3, 3a, 3b, 3c) in einer entsprechenden Achselhöhle des Benutzers (100) anordbar ist, um das Schultergelenk in einer vorbestimmten physiologischen Stellung zu halten, wobei das Stützkissen (3, 3a, 3b, 3c) eine Form aufweist, die der Form der Achselhöhle entspricht.

2. Orthopädische Vorrichtung (1) nach Anspruch 1, weiterhin umfassend ein mattenartiges Element (4), das zwischen dem Stützkissen (3, 3a, 3b, 3c) und dem Schultergurt (21) angeordnet ist, wobei das Stützkissen (3, 3a, 3b, 3c) auf dem mattenartigen Element befestigt ist.

3. Orthopädische Vorrichtung (1) nach Anspruch 2, wobei das Stützkissen (3, 3a, 3b, 3c) lösbar, besonders mittels eines Haken-/Ösen Befestigungsmittels, auf dem mattenartigen Element angeordnet ist.

4. Orthopädische Vorrichtung (1) nach Anspruch 2 oder 3, wobei das mattenartige Element (4) eine erste Oberfläche (41) aufweist, welche an der Achselhöhle des Benutzers (100) anliegt und an der das Stützkissen (3, 3a, 3b,3c) befestigt ist, und eine zweite Oberfläche (42) aufweist, die gegenüberliegend der ersten Oberfläche (41) angeordnet ist, welche am Schultergurt (21) anliegt.

5. Orthopädische Vorrichtung (1) nach einem der Ansprüche 2 bis 4, wobei das mattenartige Element (4) verschiebbar auf dem Schultergurt (21) angeordnet ist, wobei das mattenartige Element (4) auf dem Schultergurt (21) mittels eines Schlaufenabschnitts (9) befestigt ist, der dazu ausgebildet ist, den Schultergurt (21) verschiebbar aufzunehmen.

6. Orthopädische Vorrichtung (1) nach Anspruch 5, wobei eine Seitenwandung des Schlaufenabschnitts (9) mit einer Vielzahl von fensterartigen Öffnungen (91) versehen ist.

7. Orthopädische Vorrichtung (1) nach einem der Ansprüche 2 bis 6, wobei eine Vorrichtung das mattenartige Element (4) und das Stützkissen (3, 3a, 3b, 3c) umfasst und von einer Ummantelung (8) umgeben ist.

8. Orthopädische Vorrichtung (1) nach einem der Ansprüche 2 bis 7, umfassend eine Vielzahl von transversalen Versteifungselementen (6), welche mit dem mattenartigen Element (4) verbunden sind, wobei das mattenartige Element (4) mit einer Schutzhülle (5) beschichtet bzw. von dieser umgeben ist und wobei die transversalen Versteifungselemente (6) in entsprechende Taschen (7), die auf der Schutzhülle (5) ausgebildet sind, angeordnet sind.

9. Orthopädische Vorrichtung (1) nach einem der Ansprüche 2 bis 8, wobei das mattenartige Element (4) aus einem Material mit einer Gewebestruktur, in welcher eine ölige Substanz eingeschlossen ist, ausgebildet ist.

10. Orthopädische Vorrichtung (1) nach einem der Ansprüche 1 bis 9, wobei das Stützkissen (3, 3a, 3b, 3c) lösbar mit dem Schultergurt (21) verbunden ist.

11. Orthopädische Vorrichtung (1) nach einem der Ansprüche 1 bis 10, wobei der Schultergurt (21) durch Festnähen am Rückenteilabschnitt (20) befestigt ist und wobei, in einer erweiterten Ausbildung der Stützbandage (2), der Schultergurt (21) einen vorbestimmten Winkel (α) mit dem Rückenteilabschnitt (20) ausbildet und der genähte Bereich des Rückenteilabschnitts (20) in einem mit Zugkraft beaufschlagten Zustand ist.

12. Orthopädische Vorrichtung (1) nach einem der Ansprüche 1 bis 11, weiterhin umfassend einen Gurtabschnitt (23), der mit dem Rückenteilabschnitt (20) verbunden ist und der derart ausgebildet ist, um die Taille eines Benutzers (100) zu umgeben.

13. Orthopädische Vorrichtung (1) nach einem der Ansprüche 1 bis 12, weiterhin umfassend einen zweiten Schultergurt (22), welcher mit dem Rückenteilabschnitt (20) verbunden ist und der derart ausgebildet ist, um um eine Schulter des Benutzers (100) gewickelt zu werden.

14. Orthopädische Vorrichtung (1) nach Anspruch 13, umfassend ein zweites mattenartiges Element (40), welches mit dem zweiten Schultergurt (22) verbunden ist und derart ausgebildet ist, um zwischen dem zweiten Schultergurt (22) und dem Körper des Benutzers (100) angeordnet zu werden.

15. Orthopädischer Bausatz, umfassend eine orthopädische Vorrichtung (1) nach einem der Ansprüche 1 bis 14 und einen Satz Stützkissen (3a, 3b, 3c), welche sich in Form und/oder Größe voneinander unterscheiden, wobei die Stützkissen (3a, 3b, 3c) miteinander und auf dem zumindest einem Schultergurt (21) austauschbar sind.

## Revendications

1. Dispositif orthopédique (1) comprenant :
- un harnais (2) prévu avec une partie de pièce dorsale (20) adaptée pour être agencée sur le dos d'un utilisateur (100),
- au moins une bretelle (21) associée avec ladite partie de pièce dorsale (20) et adaptée pour envelopper une épaule respective de l'utilisateur (100), et
- un rembourrage de support (3, 3a, 3b, 3c) adapté pour être associé avec ladite au moins une bretelle (21),
**caractérisé en ce que** :
ledit rembourrage de support (3, 3a, 3b, 3c) est prévu pour être logé dans une cavité axillaire respective de l'utilisateur (100) pour maintenir l'articulation de l'épaule dans une position physiologique prédéterminée, dans lequel ledit rembourrage de support (3, 3a, 3b, 3c) a une forme correspondant à la forme de la cavité axillaire.

2. Dispositif orthopédique (1) selon la revendication 1, comprenant en outre un élément formant tapis (4) intercalé entre le rembourrage de support (3, 3a, 3b, 3c) et la bretelle (21), dans lequel le rembourrage de support (3, 3a, 3b, 3c) est monté sur ledit élément formant tapis (4).

3. Dispositif orthopédique (1) selon la revendication 2, dans lequel le rembourrage de support (3, 3a, 3b, 3c) est monté de manière amovible sur l'élément formant tapis (4), en particulier par des moyens de fixation à Velcro.

4. Dispositif orthopédique (1) selon la revendication 2 ou 3, dans lequel l'élément formant tapis (4) a une première face (41) prévue pour faire face à l'aisselle de l'utilisateur (100) et sur laquelle le rembourrage de support (3, 3a, 3b, 3c) est monté, et une seconde face (42) opposée à la première face (41) qui fait face à la bretelle (21).

5. Dispositif orthopédique (1) selon l'une quelconque des revendications 2 à 4, dans lequel l'élément formant tapis (4) est monté de manière coulissante sur la bretelle (21), ledit élément formant tapis (4) étant monté sur la bretelle (21) au moyen d'une partie de fourreau (9) qui est adaptée pour recevoir de manière coulissante ladite bretelle (21).

6. Dispositif orthopédique (1) selon la revendication 5, dans lequel une paroi latérale de ladite partie de fourreau (9) est prévue avec une pluralité d'ouvertures de fenêtre (91).

7. Dispositif orthopédique (1) selon l'une quelconque des revendications 2 à 6, dans lequel un ensemble comprenant l'élément formant tapis (4) et le rembourrage de support (3, 3a, 3b, 3c) est enfermé par une enveloppe (8).

8. Dispositif orthopédique (1) selon l'une quelconque des revendications 2 à 7, comprenant une pluralité d'éléments de renforcement transversaux (6) associés avec l'élément formant tapis (4), l'élément formant tapis (4) étant recouvert par une gaine de protection (5) et les éléments de renforcement transversaux (6) étant insérés dans des poches (7) respectives réalisées sur ladite gaine de protection (5).

9. Dispositif orthopédique (1) selon l'une quelconque des revendications 2 à 8, dans lequel l'élément formant tapis (4) est réalisé avec un matériau ayant une structure en treillis dans laquelle une substance huileuse est incluse.

10. Dispositif orthopédique (1) selon l'une quelconque des revendications 1 à 9, dans lequel le rembourrage de support (3, 3a, 3b, 3c) peut être associé de manière amovible avec la bretelle (21).

11. Dispositif orthopédique (1) selon l'une quelconque des revendications 1 à 10, dans lequel la bretelle (21) est fixée à la partie de pièce dorsale (20) par couture et dans lequel, dans une configuration étendue du harnais (2), la bretelle (21) forme un angle (α) prédéterminé avec la partie de pièce dorsale (20) et la région de couture de la partie de pièce dorsale (20) est dans une condition tendue par traction.

12. Dispositif orthopédique (1) selon l'une quelconque des revendications 1 à 11, comprenant en outre une partie de ceinture (23) associée avec ladite partie de pièce dorsale (20) et adaptée pour envelopper la taille de l'utilisateur (100).

13. Dispositif orthopédique (1) selon l'une quelconque des revendications 1 à 12, comprenant en outre une seconde bretelle (22) associée avec ladite partie de pièce dorsale (20) et adaptée pour envelopper une seconde épaule de l'utilisateur (100).

14. Dispositif orthopédique (1) selon la revendication 13, comprenant un second élément formant tapis (40) associé avec la seconde bretelle (22) et prévu pour être intercalé entre la seconde bretelle (22) et le corps de l'utilisateur (100).

15. Kit orthopédique comprenant un dispositif orthopédique (1) selon l'une quelconque des revendications 1 à 14 et un ensemble de rembourrages de support (3a, 3b, 3c) différents les uns des autres du point de vue de la forme et/ou de la taille, lesdits rembourrages de support (3a, 3b, 3c) étant interchangeables les uns par rapport aux autres sur ladite au moins une bretelle (21).
